# FASCICULE DE BREVET EUROPEEN

(11) **EP 1 140 119 B1**
(45) Date de publication et mention de la délivrance du brevet: **18.08.2004**
(21) Numéro de dépôt: 99958308.1
(22) Date de dépôt: 13.12.1999
(51) Int. Cl.: A61K 31/727, A61P 25/00

(54) **Utilisation des heparINES DE BAS POIDS MOLECULAIRE pour la prevention et/ou le traitement des maladies motoneuronales**
VERWENDUNG von NIEDERMOLEKULAREM HEPARIN zur Behandlung und/oder Prophylaxe motoneuronaler Krankheiten
Use OF LOW MOLECULAR WEIGHT HEPARIN for the prevention and/or treatment of motoneuronal diseases

(30) Priorité: 17.12.1998 FR 9815919
(43) Date de publication de la demande: 10.10.2001
(73) Titulaire: Aventis Pharma S.A., 92160 Antony (FR)
(72) Inventeur: STUTZMANN, Jean-Marie, F-94440 Villecresnes (FR); UZAN, André, F-75116 Paris (FR)
(74) Mandataire: Rousseau, Pierrick Edouard
(86) Numéro de dépôt international: PCT/FR1999/003109
(87) Numéro de publication internationale: WO 2000/035462

(56) Documents cités:
- WO-A-91/06303
- C. HOPF ET AL.: "Heparin inhibis acetylcholine receptor aggregation at two distinct steps in thre agrin-induced pathway." EUR. J. NEUROSCI., vol. 9, no. 6, 1997, pages 1170-1177, XP002112401
- T.H. OH ET AL.: "A muscle derived substrate-bound factor that promotes neurite outgowth from neurons of the central and peripheral nervous systems." DEV. BIOL., vol. 127, no. 1, 1988, pages 88-98, XP002112402
- "the merck index" 1996 , MERCK & CO. XP002138267 alinéa [4685]

## Description

La présente invention concerne l'utilisation des héparines de bas poids moléculaire ayant un poids moléculaire moyen compris entre 1000 et 10000 daltons dans la prévention et/ou le traitement des maladies motoneuronales.

L'héparine standard est un polysaccharide sulfaté de poids moléculaire moyen de 12000-15000 daltons isolé des muqueuses intestinales de boeuf, de mouton et de porc. L'héparine est utilisée cliniquement pour la prévention et le traitement des désordres thromboemboliques mais cause parfois des hémorragies.

Depuis une dizaine d'années, l'héparine est progressivement remplacée par les héparines de bas poids moléculaire qui ne présentent plus ou à un degré moindre l'inconvénient de faire saigner et ne nécessitent plus qu'une injection par jour au lieu de 2 à 3 injections par jour pour l'héparine standard. Ces héparines de bas poids moléculaire sont préparées notamment par fractionnement, dépolymérisation controlée de l'héparine ou par synthèse chimique. Elles présentent un rapport activité anti Xa/activité anti IIa supérieur à 2. La demande internationale WO 9106303 (Univ Case Western Reserve ; Gliatech Inc (US)) décrit l'utilisation de l'héparine ou d'une partie de la molécule composée au moins d'une unité disaccharidique pour l'inhibition de la croissance ou de la régénération nerveuse.

Il a maintenant été trouvé que les héparines de bas poids moléculaire ayant un poids moléculaire moyen compris entre 1000 et 10000 daltons augmentent la survie et/ ou la croissance des motoneurones et peuvent ainsi être utilisées dans la prévention et/ou le traitement des maladies motoneuronales.

Les maladies motoneuronales incluent la sclérose latérale amyotrophique, l'atrophie musculaire spinale progressive, l'atrophie musculaire infantile, la sclérose latérale primaire.

Selon l'invention, on utilise une héparine de bas poids moléculaire ayant un poids moléculaire moyen compris entre 1000 et 10000 daltons, notamment entre 1500 et 6000 daltons et, en particulier, entre 4000 et 5000 daltons.

Elles peuvent être préparées par différents procédés à partir de l'héparine :
- fractionnement au moyen de solvants (FR2440376, US 4692435),
- fractionnement sur résine anionique (FR2453875),
- gel filtration (BARROWCLIFFE, Thromb. Res. 12,27-36 (1977),
- chromatographie d'affinité (US4401758),
- dépolymérisation controlée au moyen d'un agent chimique : acide nitreux (EP 14184, EP37319, EP76279, EP623629, FR2503714, US4804652; WO813276), β-élimination à partir d'un ester de l'héparine (EP40144, US5389618), périodate (EP287477), borohydrure de sodium (EP347588, EP380943), acide ascorbique (US 4533549); peroxyde d'hydrogène (US4629699, US4791195), hydroxyde d'ammonium quaternaire à partir d'un sel d'ammonium quaternaire d'héparine (US4981955), hydroxyde de métal alcalin (EP380943, EP347588) ou par voie enzymatique (EP64452, US4396762, EP244235, EP244236; US 4826827; US 3766167) ou au moyen d'irradiation (EP 269981).

Certaines peuvent également être préparées par synthèse chimique (US4801583, US4818816, EP165134, EP84999, FR2535306).

Parmi ces héparines de bas poids moléculaire ayant un poids moléculaire moyen compris entre 1000 et 10000 daltons, on peut citer plus particulièrement l'énoxaparine (DCI) commercialisée par RHONE-POULENC RORER, la nadroparine (DCI) commercialisée par SANOFI, la pamaparine (DCI) commercialisée par OPOCRIN-ALFA, la reviparine (DCI) commercialisée par KNOLL, la daltéparine (DCI) commercialisée par KABI PHARMACIA, la tinzaparine (DCI) commercialisée par NOVO NORDISK, la danaparoide (DCI) commercialisée par ORGANON, l'ardeparine (DCI) développée par WYETH AYERST, la certoparine (DCI) commercialisée par SANDOZ et les produits en étude tels que le CY222 de SANOFI-CHOAY (Thromb. Haemostasis, 58 (1), 553 (1987)), le SR90107/ORG31540 de SANOFI-ORGANON (Thrombosis and Haemostasis, 74, 1468-1473 (1995)).

Préférentiellement, les héparines de bas poids moléculaire, ayant un poids moléculaire moyen compris entre 1000 et 10000 daltons sont constituées d'oligosaccharides ayant un acide 2-O-sulfo-4-énopyranosuronique à l'une de leurs extrémités.

Une héparine de bas poids moléculaire particulièrement avantageuse est obtenue par dépolymérisation d'un ester de l'héparine et, notamment, un ester benzylique, au moyen d'une base telle que la soude.

En présence de support trophique fourni par les facteurs neurotrophiques BDNF ou NT5, les cultures de motoneurones sont composées de neurones larges et homogènes avec de longs neurites branchés. Cependant, les motoneurones meurent par apoptose si la culture est effectuée en absence de support trophique.

L'effet des héparines de bas poids moléculaire ayant un poids moléculaire moyen compris entre 1000 et 10000 daltons a donc été déterminé dans un modèle de dégénération induite par privation de facteur neurotrophique de motoneurones en culture.

Par ailleurs, les astrocytes jouent un rôle majeur dans le contrôle et la maintenance d'un environnement adéquat pour la survie motoneuronale.

L'effet des héparines de bas poids moléculaire ayant un poids moléculaire moyen compris entre 1000 et 10000 daltons a ainsi également été testé sur une co-culture de motoneurones et d'astrocytes.

Les protocoles utilisés sont les suivants :

### CULTURES ENRICHIES EN MOTONEURONES :

Les cultures enrichies en motoneurones sont préparées en utilisant la méthode de centrifugation décrite par R.L. SCHNAAR et A.E. SCHAFFNER, J. Neurosci., 1, 204-217 (1981) et modifiée par W. CAMU et C.E. HENDERSON, J. Neurosci. Methods, 44, 59-70 (1992). Des moelles épinières d'embryons de rat E15 sont disséquées stérilement et débarassées des cordes spinales dorsales. Elles sont ensuite coupées et incubées 15 minutes à 37°C dans du PBS (phosphate buffer saline : NaCl 137 mM, Kcl 2,68 mM, Na₂HPO₄ 6,45 mM, KH₂PO₄ 1,47 mM) auquel on a ajouté 0,05% de trypsine. La dissociation des cellules est complétée par trituration avec l'extrémité d'une pipette de 1 ml dans le milieu de culture additionné d'albumine de sérum bovin (BSA) et de ADNase. La suspension de cellules est étalée sur une bande de métrizamide 6,5% poids/volume dans du milieu L15 (commercialisé par Gibco BRL) et centrifugée à 500 g pendant 15 minutes. La bande de l'interface contenant les motoneurones est récupérée. Les motoneurones sont étalés à une densité de 5000 cellules pour 35 mm dans des boites de culture préenduites avec de la polyomitine-laminine dans du milieu L15 auquel on a ajouté du bicarbonate de sodium (22mM), de la coalbumine (0,1 mg/ml), de la putrescine (0,1 mM), de l'insuline (5 µg/ml), du sélénite de sodium (31 nM), du glucose (20 mM), de la progestérone (21 nM), de la pénicilline (100 Ul/ml) et de la streptomycine (100 ug/ml). Les cultures sont maintenues à 37°C dans une atmosphère humidifiée à 5% de CO₂.

### CULTURE D'ASTROCYTES DE MOELLE EPINIERE :

Les astrocytes sont obtenus à partir d'embryons de rats selon la méthode de R.P. SANETO ET J. DE VELLIS, in Neurochemistry, a practical approach (A.J. TURNER et H.S. St JOHN). IRL Press, Oxford-Washington DC, p27-63 (1987) légérement modifiée. Les moelles épinières sont disséquées stérilement, débarassées des méninges et des ganglions dorsaux. Cinq à dix moelles épinières sont transférées dans du PBS (phosphate buffer saline : NaCl 137 mM, Kcl 2,68 mM, Na₂HPO₄ 6,45 mM, KH₂PO₄ 1,47 mM) et coupées avant incubation à 37°C pendant 25 minutes dans du PBS auquel on a ajouté 0,25% de trypsine. Le traitement enzymatique est stoppé par addition de 10 ml de milieu Dubelcco-Eagle modifié (DMEM) auquel on a ajouté 10% de sérum foetal de veau (FCS) et les cellules sont collectées par centrifugation. Une autre étape de dissociation mécanique est effectuée en utilisant l'extrémité d'une pipette de 1 ml. Les cellules sont étalées à une densité de 1,5-2x10⁶ cellules pour 25 cm² de milieu de culture dans du DMEM à 10% de FCS. Après 2 jours in vitro les cultures sont alimentées chaque jour tout au long de la durée de l'étude. Quand une monocouche visible de cellules est obtenue, les cultures sont agitées 48 heures à 250 rpm et, le lendemain, les monocouches sont traitées par du cytosine arabinoside (10⁻⁵ M) pendant 48 heures. Les monocouches d'astrocytes sont ensuite amplifiées à une densité de cinq pour 35 mm sur des plaques de culture pour des flacons de culture de 25 cm² au début de l'étude.

Les cultures d'astrocytes spinaux sont composées à plus de 98% de cellules immunoréactives pour la protéine gliale fibrilaire acide (GFAP). Les monocouches d'astrocytes sont exposées au produit à tester en solution dans l'eau pendant 24 heures à la concentration indiquée. Les monocouches d'astrocytes sont ensuites lavées avec du DMEM et maintenues 2 heures avec du milieu de culture où les motoneurones sont ajoutés. Deux heures après l'alimentation et durant 2 ou 3 jours, le véhicule ou le produit à tester est encore ajouté au milieu de culture.

### IMMUNOCHIMIE

Les cellules sont fixées dans 4% de paraformaldéhyde et 0, 1 % de glutaraldéhyde dans du PBS (pH 7,4 à 4°C pendant 15 minutes). Les cultures sont ensuite lavées et les sites non spécifiques bloqués avec 10% de sérum de chèvre et 2 % d'albumine de sérum bovin (BSA) dans du PBS. Ces cultures sont successivement incubées avec des anticorps de facteur de transcription Islet ½ une nuit à 4°C et de streptavidenperoxydase (1/200, Gibco) pendant 60 minutes. Les anticorps sont visualisés en utilisant la réaction DAB/peroxyde d'hydrogène. Les anticorps d'antineurofilaments (LC Amersham) sont utilisés pour l'identification des neurites.

### COMPTAGE DES CELLULES ET ANALYSE STATISTIQUE

Les cellules immunoréactives pour l'homoprotéine Islet ½ ou pour les neurofilaments et exhibant des neurites plus longs que les diamètres de 10 cellules sont considérés comme des motoneurones viables. Le nombre de motoneurones est évalué par comptage des cellules marquées dans une surface de 1,44 cm² sous microscope grossissant 200 fois. Les valeurs sont exprimées comme un nombre de motoneurones par cm² ou un pourcentage du nombre de motoneurones présents dans les cultures maintenues avec des facteurs trophiques (BDNF/NT5 1ng/mg). Les expériences sont réalisées au moins 3 fois.

Les analyses statistiques sont effectuées en utilisant le test de Student (t-test).

Les essais ont été faits en utilisant l'enoxaparine comme héparine de bas poids moléculaire.

Les résultats obtenus sont les suivants :
1 - Effet de différentes concentrations d'enoxaparine sur le nombre de motoneurones dans les co-cultures astrocytes-motoneurones

| | nombre de motoneurones % par rapport au contrôle ± écart type |
|---|---|
| véhicule | 100±21 |
| Enoxaparine | |
| 1 ng | 118±33 |
| 10 ng - | 196±47 (P<0,05) |
| 50 ng | 149±22 |

Ces résultats démontrent que le prétraitement des astrocytes avec l'enoxaparine augmente le nombre de motoneurones qui poussent sur la monocouche d'astrocytes.
Dans ce test, l'enoxaparine n'induit aucun effet morphologique apparent.
2 - Effet sur la survie motoneuronale dans les co-cultures astrocytes-motoneurones

| | survie motoneuronale % par rapport au contrôle ± écart type |
|---|---|
| véhicule | 99,9±5,1 |
| enoxaparine | |
| 1 ng/ml | 109,3±16,9 |
| 10 ng/ml⁻ | 120,7±3,2 (P=0,0066) |

Ces résultats démontrent que l'enoxaparine augmente la survie des motoneurones.
3 - effet sur le nombre de très gros motoneurones

| | nombre de gros motoneurones (500 µm) par cm³ |
|---|---|
| véhicule | 38 |
| enoxaparine | |
| 1 ng/ml | 48 |
| 10 ng/ml | 66 |

Ces résultats démontrent que l'enoxaparine augmente le nombre de gros motoneurones par rapport au contrôle.
4 - effet de potentialisation sur la stimulation de l'activité trophique motoneuronale
Les monocouches d'astrocytes répondent au stess induit par exposition à des concentrations sous-léthales de radicaux libres et augmente la production de l'activité trophique des motoneurones. En particulier les flux de faibles concentrations de peroxynitrite formé par du SIN-1 (200 umol/mn) stimulent de façon importante la capacité trophique des monocouches d'astrocytes après la fin du stimuli. L'effet de l'enoxaparine a donc été étudié sur cet effet.
Les monocouches d'astrocytes sont traitée 24 heures ave le véhicule ou l'enoxaparine (10 ng/ml) et traitées 1 heure avec 2 mM de SIN-1 (milieu azoté). Après lavage, les motoneurones sont étalés dans du milieu L15. Après 2 heures, on ajoute encore du véhicule ou de l'enoxaparine aux milieux de culture.

| | nombre de motoneurones % par rapport au contrôle |
|---|---|
| véhicule | 100 |
| SIN-1 (2mM) | 125 |
| Enoxaparine (10 ng/ml) | 115 |
| enoxaparine (10 ng/ml) + SIN-1 (2mM) | 160 |

Ces résultats démontrent que l'enoxaparine et SIN-1 augmentent la capacité trophique des astrocytes. Par ailleurs l'enoxaparine potentialise l'effet trophique du SIN-1.

La présente invention concerne l'utilisation d'une héparine de bas poids moléculaire ayant un poids moléculaire moyen compris entre 1000 et 10000 daltons pour la préparation d'un médicament utile dans la prévention et/ou le traitement des maladies motoneuronales et notamment la sclérose latérale amyotrophique, l'atrophie musculaire spinale progressive, l'atrophie musculaire infantile, la sclérose latérale primaire.

Les médicaments sont constitués par un sel (sodium ou calcium de préférence) d'une héparine de bas poids moléculaire sous forme d'une composition dans laquelle il est associé à tout autre produit pharmaceutiquement compatible, pouvant être inerte ou physiologiquement actif. Les médicaments selon l'invention peuvent être employés par voie intraveineuse, sous-cutanée, orale, rectale, topique ou pulmonaire (inhalation).

Les compositions stériles pour administration intraveineuse ou sous-cutanée, sont généralement des solutions aqueuses. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation. Elles peuvent également être préparées sous forme de compositions solides stériles qui peuvent être dissoutes au moment de l'emploi dans de l'eau stérile ou tout autre milieu stérile injectable.

Comme compositions solides pour administration orale, peuvent être utilisés des comprimés, des pilules, des poudres (capsules de gélatine, cachets) ou des granulés. Dans ces compositions, le principe actif est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un agent favorisant l'absorption orale, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

Les doses dépendent de l'effet recherché, de la durée du traitement et de la voie d'administration utilisée; elles sont généralement comprises entre 0,2 mg et 4 mg par kg par jour par voie sous-cutanée soit 14 à 280 mg par jour pour un adulte.

D'une façon générale, le médecin déterminera la posologie appropriée en fonction de l'âge, du poids et de tous les autres facteurs propres au sujet à traiter.

L'invention concerne également le procédé de préparation de médicaments utiles pour la survie et/ou la croissance des motoneurones et, en particulier, dans la prévention et/ou le traitement de maladies motoneuronales et, notamment la sclérose latérale amyotrophique, l'atrophie musculaire spinale progressive, l'atrophie musculaire infantile, la slérose latérale primaire, consistant à mélanger une héparine de bas pois moléculaire ayant un poids moléculaire moyen compris entre 1000 et 10000 daltons avec un ou plusieurs diluants et/ou adjuvants compatibles et pharmaceutiquement acceptables.

## Revendications

1. Utilisation d'une héparine de bas poids moléculaire, ayant un poids moléculaire moyen compris entre 1000 et 10000 daltons, pour la préparation d'un médicament pour la prévention et/ou le traitement des maladies motoneuronales.

2. Utilisation selon la revendication 1 pour la prévention et/ou le traitement de la sclérose latérale amyotrophique, l'atrophie musculaire spinale progressive, l'atrophie musculaire infantile, la slérose latérale.

3. Utilisation selon l'une des revendications 1 à 2 pour laquelle l'héparine de bas poids moléculaire a un poids moléculaire moyen compris entre 1500 et 6000 daltons.

4. Utilisation selon l'une des revendications 1 à 2 pour laquelle l'héparine de bas poids moléculaire a un poids moléculaire moyen compris entre 4000 et 5000 daltons.

5. Utilisation selon l'une des revendications 1 à 4 pour laquelle l'héparine de bas poids moléculaire est constituée d'oligosaccharides ayant un acide 2-O-sulfo-4-énopyranosuronique à l'une de leurs extrêmités.

6. Utilisation selon l'une des revendications 1 à 5 pour laquelle l'héparine de bas poids moléculaire est obtenue par dépolymérisation d'un ester de l'héparine au moyen d'une base.

7. Utilisation selon l'une des revendications 1 à 6 pour laquelle l'héparine de bas poids moléculaire est l'énoxaparine.

8. Utilisation selon l'une des revendications 1 à 4 pour laquelle l'héparine de bas poids moléculaire la nadroparine.

9. Utilisation selon l'une des revendications 1 à 4 pour laquelle l'héparine de bas poids moléculaire est la parnaparine.

10. Utilisation selon l'une des revendications 1 à 4 pour laquelle l'héparine de bas poids moléculaire est la reviparine.

11. Utilisation selon l'une des revendications 1 à 5 pour laquelle l'héparine de bas poids moléculaire est la daltéparine.

12. Utilisation selon l'une des revendications 1 à 4 pour laquelle l'héparine de bas poids moléculaire est la tinzaparine.

13. Utilisation selon l'une des revendications 1 à 6 pour laquelle l'héparine de bas poids moléculaire est la danaparoide.

14. Utilisation selon l'une des revendications 1 à 4 pour laquelle l'héparine de bas poids moléculaire est l'ardeparine.

15. Utilisation selon l'une des revendications 1 à 4 pour laquelle l'héparine de bas poids moléculaire est la certoparine.

16. Utilisation selon l'une des revendications 1 à 4 pour laquelle l'héparine de bas poids moléculaire est le CY222.

17. Utilisation selon l'une des revendications 1 à 4 pour laquelle l'héparine de bas poids moléculaire est le SR90107/ORG31540.

## Patentansprüche

1. Verwendung eines Heparins mit niedrigem Molekulargewicht, das ein mittleres Molekulargewicht zwischen 1000 und 10000 Dalton hat, für die Herstellung eines Medikaments zur Vorbeugung und/oder Behandlung der motoneuralen Erkrankungen.

2. Verwendung gemäß Anspruch 1 für die Vorbeugung und/oder Behandlung der amyotrophischen Lateralsklerose, der progressiven spinalen Muskelatrophie, der infantilen Muskelatrophie, der Lateralsklerose.

3. Verwendung gemäß einem der Ansprüche 1 bis 2, für die das Heparin mit niedrigem Molekulargewicht ein mittleres Molekulargewicht zwischen 1500 und 6000 Dalton hat.

4. Verwendung gemäß einem der Ansprüche 1 bis 2, für die das Heparin mit niedrigem Molekulargewicht ein mittleres Molekulargewicht zwischen 4000 und 5000 Dalton hat.

5. Verwendung gemäß einem der Ansprüche 1 bis 4, für die das Heparin mit niedrigem Molekulargewicht aus Oligosacchariden besteht, die eine 2-O-Sulfo-4-enopyranosuronsäure an einem ihrer Enden haben.

6. Verwendung gemäß einem der Ansprüche 1 bis 5, für die das Heparin mit niedrigem Molekulargewicht durch Depolymerisation eines Esters des Heparins mittels einer Base erhalten wird.

7. Verwendung gemäß einem der Ansprüche 1 bis 6, für die das Heparin mit niedrigem Molekulargewicht Enoxaparin ist.

8. Verwendung gemäß einem der Ansprüche 1 bis 4, für die das Heparin mit niedrigem Molekulargewicht Nadroparin ist.

9. Verwendung gemäß einem der Ansprüche 1 bis 4, für die das Heparin mit niedrigem Molekulargewicht Parnaparin ist.

10. Verwendung gemäß einem der Ansprüche 1 bis 4, für die das Heparin mit niedrigem Molekulargewicht Reviparin ist.

11. Verwendung gemäß einem der Ansprüche 1 bis 5, für die das Heparin mit niedrigem Molekulargewicht Dalteparin ist.

12. Verwendung gemäß einem der Ansprüche 1 bis 4, für die das Heparin mit niedrigem Molekulargewicht Tinzaparin ist.

13. Verwendung gemäß einem der Ansprüche 1 bis 6, für die das Heparin mit niedrigem Molekulargewicht Danaparoid ist.

14. Verwendung gemäß einem der Ansprüche 1 bis 4, für die das Heparin mit niedrigem Molekulargewicht Ardeparin ist.

15. Verwendung gemäß einem der Ansprüche 1 bis 4, für die das Heparin mit niedrigem Molekulargewicht Certoparin ist.

16. Verwendung gemäß einem der Ansprüche 1 bis 4, für die das Heparin mit niedrigem Molekulargewicht CY222 ist.

17. Verwendung gemäß einem der Ansprüche 1 bis 4, für die das Heparin mit niedrigem Molekulargewicht SR90107/ORG31540 ist.

## Claims

1. Use of a low molecular weight heparin, having a mean molecular weight of between 1000 and 10 000 daltons, for preparing a medicinal product for the prevention and/or treatment of motoneuron diseases.

2. Use according to Claim 1, for the prevention and/or treatment of amyotrophic lateral sclerosis, progressive spinal muscular atrophy, infantile muscular atrophy and lateral sclerosis.

3. Use according to either of Claims 1 and 2, for which the low molecular weight heparin has a mean molecular weight of between 1 500 and 6 000 daltons.

4. Use according to either of Claims 1 and 2, for which the low molecular weight heparin has a mean molecular weight of between 4 000 and 5 000 daltons.

5. Use according to one of Claims 1 to 4, for which the low molecular weight heparin consists of oligosaccharides having a 2-O-sulpho-4-enopyranosuronic acid at one of their ends.

6. Use according to one of Claims 1 to 5, for which the low molecular weight heparin is obtained by depolymerization of a heparin ester using a base.

7. Use according to one of Claims 1 to 6, for which the low molecular weight heparin is enoxaparin.

8. Use according to one of Claims 1 to 4, for which the low molecular weight heparin is nadroparin.

9. Use according to one of Claims 1 to 4, for which the low molecular weight heparin is parnaparin.

10. Use according to one of Claims 1 to 4, for which the low molecular weight heparin is reviparin.

11. Use according to one of Claims 1 to 5, for which the low molecular weight heparin is dalteparin.

12. Use according to one of Claims 1 to 4, for which the low molecular weight heparin is tinzaparin.

13. Use according to one of Claims 1 to 4, for which the low molecular weight heparin is danaparoid.

14. Use according to one of Claims 1 to 4, for which the low molecular weight heparin is ardeparin.

15. Use according to one of Claims 1 to 4, for which the low molecular weight heparin is certoparin.

16. Use according to one of Claims 1 to 4, for which the low molecular weight heparin is CY222.

17. Use according to one of Claims 1 to 4, for which the low molecular weight heparin is SR90107/ORG31540.
